Europäisches Patentamt

(19) European Patent Office

Office européen des brevets

(11) Publication number : 0 503 943 A1

(12) **EUROPEAN PATENT APPLICATION**

(21) Application number : 92302131.5

(22) Date of filing : 12.03.92

(51) Int. Cl.[5] : **C12Q 1/00,** G01N 33/94

(30) Priority : 14.03.91 GB 9105406

(43) Date of publication of application :
16.09.92 Bulletin 92/38

(84) Designated Contracting States :
PT

(71) Applicant : IMPERIAL CHEMICAL
INDUSTRIES PLC
Imperial Chemical House, Millbank
London SW1P 3JF (GB)

(72) Inventor : Vadgama, Pankaj Madganlal
4 The Sidings, Drywood Avenue
Worsley, Manchester M28 4QA (GB)
Inventor : Rosenberg, Mark Franklyn
5 Castle Hill Road
Prestwich, Manchester M25 8FR (GB)
Inventor : Christie, Ian McIntyre
23 Macclesfield Road, Whaley Bridge
Stockport, Cheshire SK12 7DG (GB)

(74) Representative : Tunnicliffe, Peter Barry
Westfields House 5 Vincent Drive Lache Lane
Chester, CH4 7RQ (GB)

(54) Sensor devices.

(57) Analytical sensor devices incorporating a permselective medium immobilised as a continuous liquid or gel phase barrier between sample and detector.

The barrier is electrically insulating, impermeable to charged non-lipophilic species, selectively partitions lipophilic species diffusing through it, and its selectivity can be controlled or optimised by pH adjustments. The preferred barrier form has the permselective medium (e.g. a lipid or ester) immobilised by incorporation as plasticiser in a polymer (especially PVC). The preferred detector is an electrode, especially of non-potentiometric type.

The sensor can be used to detect components as such or indirectly by allowing them to react to produce other materials to which the detector responds (e.g. an ionic species or an ion concentration or activity change) and is also applicable to detecting neutral or small uncharged hydrophilic species, e.g. hydrogen peroxide – especially that formed by enzyme action in the sensor or detector system.

FIG.1

EP 0 503 943 A1

Jouve, 18, rue Saint-Denis, 75001 PARIS

# SENSOR DEVICES

This invention relates to sensor devices such as are used in the determination of a component or components which may be present in a fluid sample such as a physiological fluid (eg. blood) or an effluent.

In general, the component to be assayed will be lipophilic and, in addition to the component to be determined, the fluid samples to be analysed will contain other components which, in the absence of selectivity, will interfere with the determination of the desired component. Typically, the fluid samples to be analysed will consist predominantly of hydrophilic components and ionised species. For example, in the case of physiological fluids, the lipophilic component to be determined may be a phenolic compound such as paracetemol (acetaminophen), and interferents commonly encountered in analysis of physiological fluids using amperometric sensors are charged ascorbate, urate, glutathione and certain amino acids. Reliable determination of the desired component therefore demands effective selectivity.

According to one aspect of the present invention there is provided a sensor device comprising means for detecting a preselected lipophilic component present in a fluid sample and providing an output representative of the content of said component in the sample, a permselective medium and means immobilising said permselective medium as a continuous liquid or gel phase to form a substantially electrically insulating barrier between the detecting means and a fluid sample to be analysed, the permselective medium being substantially impermeable to charged substantially non-lipophilic species that may be present in samples to be analysed and being selective, by partitioning, for lipophilic species whereby any of said preselected lipophilic component in the sample partitions into and diffuses through the barrier for detection on the other side of the barrier by the detecting means.

The detecting means is conveniently of an electrochemical nature but other types, such as spectrophotometric or optical detecting systems, may be employed.

The detecting means will usually comprise an electrode system and a liquid or gel phase electrolyte containing medium.

In most applications, the electrolyte medium will be aqueous but it is to be understood that, as used herein, electrolyte media is to be construed as including media which are organic-based as well as aqueous-based media. Preferably the permselective barrier is substantially impermeable to the electrolyte medium.

The preselected lipophilic component may be electrochemically active at the electrode operating conditions used in which case the detecting system is conveniently directly responsive to the preselected component. Especially where the preselected component exhibits no or only insignificant electrochemical activity, the detecting means preferably comprises means for interacting with the preselected component to produce a directly detectable electrochemically active species. Thus, for example, the detecting means may comprise an electrolyte medium incorporating an enzyme effective to interact with the preselected lipophilic component to produce a readily detectable electro-chemically active species.

According to a more specific aspect of the present invention there is provided a sensor device comprising a detecting system including an electrolyte medium, a permselective medium and means immobilising said permselective medium as a continuous liquid or gel phase to form a substantially electrically insulating barrier between the electrolyte medium and a fluid sample to be analysed, the permselective medium being substantially impermeable to charged substantially non-lipophilic species that may be present in samples to be analysed and being selective, by partitioning, for lipophilic species contained in such samples.

According to a further aspect of the invention there is provided a method for determining a lipophilic analyte in a specimen containing charged substantially non-lipophilic species, which method comprises contacting the specimen with a permselective medium which is immobilised as a continuous liquid or gel phase to form an electrically insulating barrier layer enclosing a detecting system including an electrolyte medium, such contact of the specimen with the permselective material being made at the opposite side of the barrier to that side presented towards the electrolyte medium and the permselective medium being substantially impermeable to said charged substantially non-lipophilic species and being selective, by partitioning, for said lipophilic analyte, and using the detecting system to directly or indirectly detect the lipophilic analyte diffusing through the barrier.

Preferably the detecting system comprises an electrode system but it may alternatively be in some other form such as a spectrophotometric or optical system. Where an electrode system is used, it is preferably of the non-potentiometric type. Examples of non-potentiometric electrode systems include the amperometric, galvanic, photo-galvanic and coulometric types.

The permselective medium will usually be permeable to gases, eg. oxygen.

The analyte may be detected directly, for example by using the electrode system to effect decomposition of the analyte with consequent generation of an electrode current. Thus for example, in the case of an amperometric electrode system, the working electrode may be electrically polarised so as to effect decomposition of at least one lipophilic organic species which it is desired to detect. Alternatively, the analyte may be detected by an indirect method; for example, after permeation through the barrier, the analyte may interact with a suitable enzyme on the electrode side of the barrier such that the interaction results in the production or consumption of a species which can be directly detected electrochemically.

Although the permselective medium is selective towards lipophilic species, we do not exclude the possibility of the sensor being used to detect suitable electrically neutral hydrophilic compounds, particularly hydrogen peroxide since, as described hereinafter, it is possible to employ as the permselective medium a lipophilic selective medium which, while generally substantially impermeable to charged hydrophilic species and most neutral hydrophilic compounds, may nevertheless exhibit some degree of selectivity towards a small uncharged hydrophilic species such as hydrogen peroxide such that sufficient hydrogen peroxide permeates the permselective medium to allow measurable signals to be obtained from the detecting system without significant interference from other hydrophilic species.

According to a further aspect of the invention there is provided a method for the electrolytic determination of hydrogen peroxide derived from a specimen containing charged substantially non-lipophilic species, which method comprises contacting the specimen with a permselective medium which is immobilised as a continuous liquid or gel phase to form an electrically insulating barrier layer enclosing a detecting system including an electrolyte medium, such contact of the specimen with the permselective material being made at the opposite side of the barrier to that side presented towards the electrolyte medium and the permselective medium being substantially impermeable to said charged substantially non-lipophilic species and being at least partially selective towards hydrogen peroxide, and using the detecting system to detect hydrogen peroxide diffusing through the barrier.

The hydrogen peroxide may for example be generated as a result of interaction of a desired component with an enzyme.

In one embodiment of the invention, the permselective medium may be immobilised by confinement between a pair of supports, which supports may be permeable to ions, hydrophilic and lipophilic species, eg. a pair of dialysis membranes with spacing means therebetween, the spacing means having a structure such that the permselective medium permeates the spacing means to form a continuous liquid or gel phase.

Preferably however, the permselective medium is immobilised by incorporation in a polymeric material such that the permselective medium forms a continuous phase and is preferably present throughout the thickness of the layer or film formed by the polymer and the permselective material.

The permselective medium conveniently comprises a substantially water immiscible fatty or oily substance (natural or otherwise), for example a lipid or ester.

In a preferred embodiment of the invention, the permselective medium comprises a plasticiser which is substantially miscible with the polymeric material so as to form a substantially homogeneous phase in which the plasticiser is immobilised by the polymeric compound. The plasticiser may be any one of those compounds commonly used as plasticisers in polymeric compounds provided that the plasticiser possesses the selectivity and impermeability characteristics referred to above. The permselective medium in general should also have a low volatility and good resistance to extraction into aqueous and organic phases.

Suitable compounds for use as the permselective medium, and which also have plasticiser characteristics, include di-octyl phthalate (DOP) and iso-propyl myristate (IPM). Other plasticisers such as diphenyl ether and dioctyl adipate may also be suitable.

Where the permselective medium is incorporated in a polymeric material, a suitable polymeric material is poly vinyl chloride (PVC). Thus, in a particularly advantageous form of the invention, the barrier is constituted by a layer of plasticised polymer, preferably PVC with DOP or IPM as the plasticiser.

There may be more than one barrier as aforesaid between the electrolyte medium and the fluid sample to be analysed. For example, the permselective medium may be immobilised as two or more superimposed layers, possibly with interposed media such as reagent-containing films or layers in which intermediate reactions may occur and which may contain an enzyme for this purpose, or films/layers possessing desired characteristics, eg. predetermined dielectric and/or pH characteristics. Thus, in some cases, there may be two or more superimposed films of plasticised polymeric material arranged in overlying relation with the electrode or other detecting system.

Where the permselective medium is incorporated in a polymeric material to form a film, exposure of the permselective material at the surfaces of the film may result in some loss of the permselective medium to the surroundings, eg. there may some tendency for the permselective medium to leach out of the polymeric material into the sample fluid and/or the electrolyte medium or any other material in close proximity to the film such as a rubber sealing ring or the like. The film should therefore be of sufficient thickness that any such loss of the permselective medium at the surfaces of the film is not so extensive as to fully deplete the film of permselective medium at any location over the working area of the film. In particular, the thickness of the film should be such that any depletion is not so extensive that the film becomes porous and allows species, especially charged non-lipophlic species, to transfer from one side of the film to the other through the pores. Film thicknesses no less than 5 microns, preferably no less than 10 microns, are favoured and typically the film thickness will be at least 25 microns thick.

A feature of the invention is that the use of a permselective medium which is substantially impermeable to charged non-lipophilic species allows the existence of a pH differential between opposite sides of the barrier. Thus, the pH values may in appropriate cases be adjusted to according to requirements, eg. to optimise the signals produced by the electrode system (for instance, in terms of signal magnitude and/or speed of response) and/or maintain the pH on the fluid sample side of the barrier at physiological levels for enzyme/antibody reactions, while allowing a desired reaction product to permeate to the other side of the barrier where a suitable pH level may prevail for promoting electrochemical detection of the desired product or for providing an optimum pH for an enzyme.

In some instances, the pH on the sample side of the barrier may be adjusted so as to determine the extent to which the permselective barrier is selective for certain components in the sample. For example, the lipophilicity of components may be controlled by pH adjustment of the sample so that a certain component which is lipophilic within a predetermined pH range is rendered substantially non-lipophilic thereby substantially preventing partitioning of that component into the material of the barrier (or vice versa). In this manner, where a particular lipophilic component is to form the analyte, adjustment of the pH of the sample can be used to reduce or eliminate permeation through the barrier of potentially competing components which would otherwise be highly lipophilic in the absence of such pH adjustment.

Alternatively, pH adjustment of the sample may be employed to render a substantially non-lipophilic component sufficiently lipophilic to enable it to permeate the barrier. A typical application arises in the detection of salicylate in blood where paracetemol is a competing component. At physiological pH levels, paracetemol is lipophilic but salicylate is substantially non-lipophilic. However, by adjusting the sample towards lower pH levels, the salicylate converts to salicylic acid which is lipophilic and can therefore partition into the barrier material and permeate through to the other side along with the paracetemol component. The salicylic acid can then be detected electrochemically even in the presence of paracetemol since, using amperometric detection, the former provides a strong signal compared with paracetemol.

The current conducting medium may include some form of scavenger which is effective to consume or otherwise negate the affect of any decomposition products which may be undesirable, for example decomposition products arising from the decomposition of the analyte which may adversely affect the pH conditions of the electrolyte may be compensated for by a suitable buffer.

The sensor of the invention may take various forms. For example, it may be of the enzyme type in which an enzyme such as glucose oxidase is immobilised and used to promote the oxidation of glucose in the presence of oxygen so that the hydrogen peroxide so formed (as a co-product when the glucose is oxidised to gluconic acid) can then be measured after permeation of the hydrogen peroxide through the permselective medium. Alternatively, the sensor may incorporate an enzyme within the electrolyte medium (which may employ an organic compound as solvent) such that the analyte interacts with the enzyme to produce a product which can be measured amperometrically. In the latter instance, where the analyte is ethanol for example, the enzyme may be alcohol oxidase which may be suspended or dissolved in the electrolyte medium or suitably immobilised on the electrode side of the permselective medium, the interaction between the ethanol analyte and the enzyme generating hydrogen peroxide for detection by the electrode system.

According to a further aspect of the present invention there is provided a sensor device comprising an electrode system including an ion selective electrode, a permselective medium and means immobilising said permselective medium as a continuous liquid or gel phase to form a substantially electrically insulating barrier between the ion selective electrode and a fluid sample to be analysed, the permselective medium being substantially impermeable to non-gaseous charged substantially non-lipophilic species that may be present in samples to be analysed and being selective towards lipophilic organic species contained in such samples, there being an electrolyte medium present between said barrier and said ion selective electrode, and said electrolyte medium containing an agent which interacts with at least a preselected lipophilic compound to produce an ionic species, or a concentration or activity change in an ionic species, to which the ion selective electrode is responsive.

The ion selective electrode may be of the coated wire type.

In this further aspect of the invention, the permselective medium and the means immobilising the same may have the characteristics defined above in connection with those aspects of the invention relating to non-potentiometric electrode systems, including the use of plasticised PVC as the barrier.

It is known in potentiometric electrode systems to use as the ion selective membrane a plasticised PVC membrane incorporating an ion exchanger, the ions interacting at the outer surface of the PVC membrane whereby a potential difference is established across the thickness of the ion selective membrane. In accordance with said further aspect of the present invention, plasticised PVC is used in conjunction with the ion selective membrane so as to exclude interferent species, ie. the plasticised PVC forming the barrier does not function as a ion selective membrane in the sense used in conventional ion selective electrode systems.

The electrode system generally also includes a reference electrode and both the ion selective electrode and the reference electrode are conveniently immersed in the electrolyte medium provided between the ion selective electrode and said barrier.

The invention will now be described by way of example only with reference to the accompanying drawings in which:

Figure 1 is a diagrammatic sectional view of a sensor incorporating a permselective barrier in accordance with the invention;

Figure 2 is an enlarged view of part of the sensor shown in Figure 1;

Figure 3 is a graph in the form of a histogram showing the sensitivity to certain analytes and interferents of a sensor in accordance with the invention and similar sensors using polycarbonate or cellulose acetate membrane with a covering polycarbonate membrane;

Figure 4 is a graph showing electrode response as a function of sample pH for catechol and hydrocaffeic acid;

Figures 5 and 6 are graphs showing electrode response as a function of variation in plasticiser loading in PVC, the plasticisers being DOP and IPM respectively;

Figure 7 is a diagrammatic longitudinal sectional view of an alternative sensor configuration;

Figure 8 is an end view of the sensor of Figure 7;

Figures 9 and 10 are diagrams showing different assay schemes which may be used in sensors according to the invention; and

Figures 11A and 11B are structural formulae for compounds that may be used in the scheme of Figure 10.

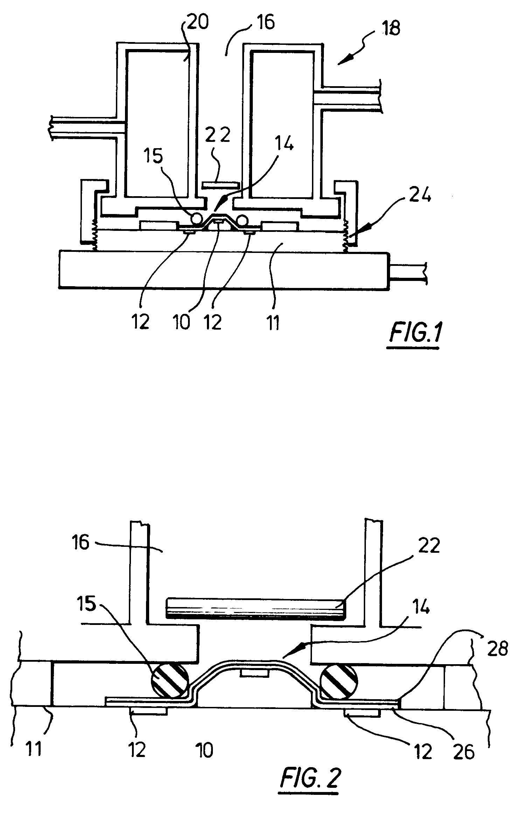

Referring to Figures 1 and 2, the sensor comprises an amperometric electrode system comprising a support 11 carrying a central platinum working electrode 10 encircled by an annular pseudo-reference electrode 12 composed of silver. A membrane structure 14 is located over the electrode system 10, 12, the membrane structure 14 being sealed by O-ring 15 against the support 11 so as to enclose an electrolyte by means of which the electrolytic contact is made between the electrodes 10 and 12. The pH of the electrolyte may differ substantially from that of the specimen to be investigated (which will usually be of an aqueous nature, eg. a physiological fluid or an aqueous effluent) since the barrier formed by the membrane is substantially impermeable to ions and charged hydrophilic compounds.

The specimen to be investigated is applied to the outer face of the membrane structure, ie. on the side of the membrane structure opposite to the electrode system. The specimen is introduced into a specimen chamber 16 defined by a structure 18 which incorporates a water jacket 20 for maintaining the specimen at a desired temperature. The chamber 16 includes a stirrer bar 22 which is rotated by suitable magnetic drive (not shown). The structure 18 includes a screw-threaded fitting 24 by means of which it can be clamped to the support 11 to compress the sealing ring 15.

The membrane structure 14 comprises two layers, a first layer 26 comprising a dialysis membrane which acts as a support and as a spacer and a second layer 28 composed of a plasticised PVC. The first layer is permeated with the electrolyte medium which therefore contacts the platinum and silver electrodes. The plasticised PVC has the property of being substantially impermeable to the electrolyte and to non-lipophilic species (both ionised and un-ionised) and it is therefore possible for the pH on opposite sides of the PVC membrane to differ substantially and for the pH differential to be maintained. The barrier properties of the PVC membrane also makes it feasible for the phase on the electrode side of the membrane (and also the specimen phase) to be non-aqueous since the PVC membrane will prevent any substantial intermixing between the phases on opposite sides of the barrier; thus, in some circumstances, the electrolyte may be dissolved in an organic solvent.

The PVC membrane is selectively permeable to lipophilic compounds, such as un-ionised phenols, and whilst its permeability with respect to certain uncharged hydrophilic compounds, particularly hydrogen peroxide, may be very limited, its permeability to such compounds as compared with charged non-lipophilic compounds is such that it is in effect selectively permeable to at least hydrogen peroxide thus allowing the sensor to be used in applications where the analyte to be determined can be measured by measurement of hydrogen peroxide derived from the analyte in the specimen.

The PVC forming the membrane layer 28 is plasticised but is substantially devoid of any ion exchange material. However, there may be circumstances where an ion exchange, or other, material is incorporated in the membrane layer 28, eg. a compound having molecular affinity with the analyte or a derivative thereof, to facilitate or enhance transport a desired component through the membrane. The plasticiser may be an oily or fatty substance such as di-octyl phthalate (DOP) or iso-propyl myristate (IPM).

EXAMPLE

PVC membranes for use in sensors according to the invention were produced by the following method. 10 ml of tetrahydrofuran (THF) was added to a beaker and plasticiser, typically IPM or DOP in quantities ranging from 50 to 300 µl, was added. 0.06g of PVC powder (MW-200,000) was then added and the mixture was stirred until the PVC was fully dissolved. A 9 cm Petri dish was rinsed with THF and the PVC solution was poured into the dish to form a thin film. The lid of the Petri dish was put in place and the dish was left in a fume cupboard for 2 to 3 days until the THF had evaporated, leaving a film of plasticised PVC on the dish, typically of the order of 25 microns thick. A section of the PVC film was removed by cutting out a suitably dimensioned area with a scalpel blade, superimposing a similarly sized section of dialysis membrane (pre-soaked in buffer solution) on that section of PVC so that the two layers adhered, and then lifting the two layers together with a peeling action initiated with the aid of a scalpel blade. The dialysis membrane used was of Cuprophan, removed from a haemodialysis cartridge.

In an alternative method, the PVC solution referred to above was cast onto a piece of dialysis membrane by using adhesive tape to form a suitably dimensioned window of dialysis material on to the exposed area of which the PVC solution was cast, e.g. 15 µl PVC solution (10 ml THF, 0.06 g PVC and 0.15 ml IPM) was pipetted onto the window. The adhesive tape in addition to defining the window, also served to confine the solution to the window area and prevented the dialysis membrane curling. After the THF had been allowed to evaporate, the dialysis membrane/PVC membrane sandwich was cut just within the boundaries of the window to allow removal of the PVC coated dialysis membrane section.

The membrane structures so produced were assembled in the manner illustrated in the drawing with a Rank (Bottisham, Cambridge) oxygen cell electrode system comprising a platinum anode and a silver cathode functioning as a pseudo-reference. Before use, the electrodes were conditioned by polarisation of the platinum electrode at +650 mV (vs Ag), in a phosphate buffer until a baseline was achieved. In assembling the cell, the electrodes were covered by a small quantity of buffer and the membrane structure (dialysis membrane/PVC membrane) was placed over the electrodes. A sample chamber with O-ring was then placed over the membrane structure, stretching the membrane over the raised anode and the sample chamber was secured to the electrode system. The sample chamber was then rinsed and filled with the sample to be investigated.

The phosphate buffer/electrolyte used typically had a pH of 7.4 and comprised 2.44g $NaH_2PO_4.4H_2O$, 7.5g $Na_2HPO_4$, 3.0g NaCl and 0.6g EDTA per litre. Measurements were made with the platinum anode polarised at +650 mV (vs Ag) after a 2 hour conditioning period at this voltage in buffer.

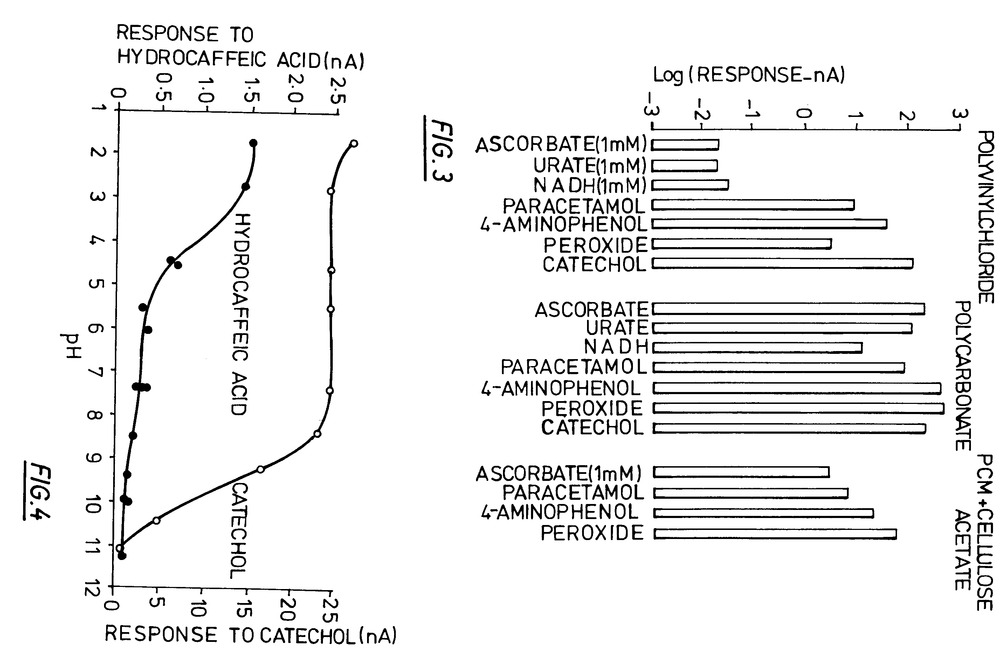

Figure 3 illustrates the selectivity achieved with plasticised PVC membranes produced in using the first of the methods described above, ie. by casting onto a Petri dish. It will be seen that the responses obtained for hydrogen peroxide and the uncharged lipophilic compounds paracetemol (acetominophen), 4-aminophenol, and catechol (used in 0.1mM solutions) are considerably greater than those obtained for the interferents ascorbate, urate and NADH (used in 1.0mM solutions in order to enhance the highly attenuated signals obtained) which are commonly encountered in clinical analysis using amperometric sensors. For comparison, Figure 3 also shows the responses obtained using other forms of membrane (polycarbonate membrane and cellulose acetate/polycarbonate membrane) commonly used in biosensors using 0.1mM solutions in all cases, except where indicated otherwise, from which it will be seen that the determinand:interference signal ratios demonstrate the very high selectivity of plasticised PVC membranes, in particular for the phenolics, over the common interferents ascorbate and urate.

The polycarbonate membrane used in deriving the middle range of responses shown in Figure 3 comprised a piece of "NUCLEPORE" polycarbonate film with a mean pore diameter of 0.05 microns. For the range of responses shown at the right hand side of Figure 3, the membrane comprised a layer of cellulose acetate (produced by casting of a 2% solution of cellulose acetate in acetone followed by evaporation of the solvent) over which a film of "NUCLEPORE" polycarbonate having a mean pore diameter of 0.03 microns is placed.

It was found that permeation through PVC is dependent on the charge carried by the molecule/ion, with consequent dependence on the pH of the specimen. Signals obtained for catechol (pKa = 9.46) and hydrocaffeic acid (carboxylic pKa approx 4.8), with varying pH of the specimen, indicated that increasing pH values, approaching the pKa value and consequent ionisation of the phenol/carboxyl group, gave substantially reduced signal size. It follows from this that to secure good selectivity towards the analyte, the pH of the specimen fluid should be sufficiently far removed from the pKa value of the analyte in order to minimise ionisation of the analyte compound and hence maximise its permeation through the PVC membrane.

Figure 4 illustrates the effect of variation in the sample pH for catechol and hydrocaffeic acid. Thus, in the case of catechol, the electrode response is approximately constant over a wide range of pH values but falls drastically at pH values in the region of pKa = 9.46. At pH levels in, and above, this region, catechol ionises and in its ionised form is substantially non-lipophilic. Similarly, it will be seen that hydrocaffeic acid exhibits similar characteristics but at different pH levels. Because the plasticised PVC film is substantially impermeable to charged non-lipophilic species, and because in practice such species will predominate, it is possible for significantly differing pH levels to co-exist on the sample and electrode sides of the film, thus enabling special effects, such as those previously described, to be obtained by selection of the pH levels prevailing on each side.

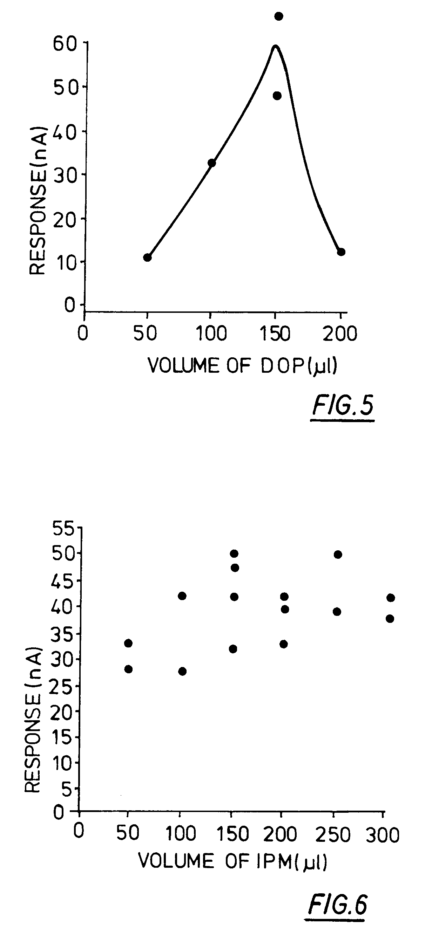

Figure 5 illustrates the effect of varying the amount of DOP in the PVC film. In the case of DOP, it will be seen that there is a pronounced peak in the electrode response indicating an optimum DOP content. In the case of IPM, variation of the IPM content did not exhibit a well-defined peak (see Figure 6).

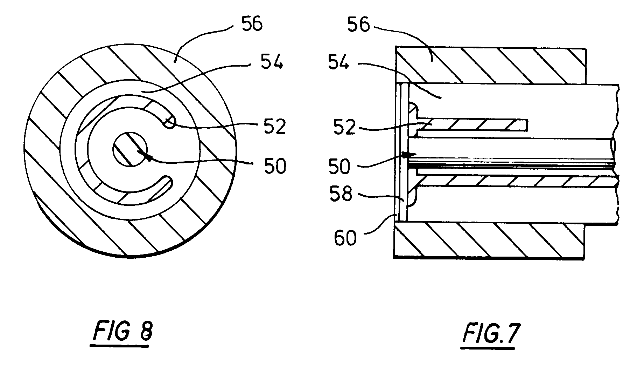

Referring now to Figures 7 and 8 of the drawings, there is shown a sensor which may be used in the manner of a dipstick. The sensor comprises an electrode system of the amperometric type with a a central platinum working electrode 50 and an encircling part annular pseudo-reference silver electrode 52, the two electrodes being embedded in a support 54 of electrically insulating material, such as epoxy resin, and arranged with the exposed end face of the electrode 50 substantially coplanar with the exposed surface of the annular electrode 52. The support 54 is enclosed within a sleeve 56 such that end face of the sleeve 56 projects beyond the exposed surfaces of the electrodes 50 and 52 to form a recess in which a layer 58 of electrolyte medium is received together with an overlying layer 60 of a permselective medium having properties in accordance with the invention.

The electrolyte medium may for example comprise a polyacrylamide gel prepared by mixing aqueous solutions of:

   300 μl 30% acrylamide (electrophoresis grade);
   100 μl 2.6% N,N'-methylene-bis-acrylamide (electrophoresis grade; and
   100 μl 2% N',N',N',N-tetra-ethyl methylene diamine.

100 μl of 2.8% potassium persulphate was added to the mixed solutions and 10 μl of the mixture was spread onto the end faces of the electrodes to form the layer 58.

The permselective layer 60 may comprise a plasticised PVC film which may be formed by spreading 40 μl of a solution of PVC containing PVC and IPM in THF over the gelled or solidified layer 58 of electrolyte medium to produce a continuous film which is then allowed to dry.

SPECIFIC APPLICATIONS

One application of the invention is in the carrying out of homogeneous immunoassays for the detection of amphetamines. Kits for this purpose are commercially available which include an anti-amphetamine anti-body (Ab), that binds to the antigen amphetamine (Ag), and an agent (Ag*) comprising amphetamine which is chemically attached to the enzyme glucose-6-phosphate dehydrogenase (G6PDH). Conventionally the assay is performed in the following manner:

The sample to be tested for amphetamine content is mixed with a suitable buffer, antibody Ab and the agent Ag*. The antibody binds to the amphetamine in the sample and also to the agent Ag* and the amount of antibody employed is such that there is excess Ag and Ag* so that some of the Ag and Ag* remains unbound. It is a feature of G6PDH that, when bound to an antibody, it is inactivated so that after binding, the only active G6PDH is part of the agent Ag*. Thus, the higher the Ag content of the sample, the lower the amount of antibody available to bind with the agent Ag* and the unbound Ag* available after incubation is therefore representative of the amount of Ag originally present in the sample, ie. greater amounts of unbound Ag* indicate higher contents of amphetamine in the original sample.

Detection of the free active Ag* is by the reaction of glucose-6-phosphate (G6P) and NAD+ (β-nicotinamide adenine dinucleotide) to yield inter alia NADH (reduced β-nicotinamide adenine dinucleotide). Conventionally, the kit assay then requires spectrophotometric detection of the NADH in order to derive a measure of the amphetamine in the original sample.

The present invention allows an alternative approach to spectrophotometric detection. In order to yield an electroactive species suitable for use in a sensor according to the invention, the sample is processed in the manner described above but with the inclusion, in the reaction mixture, of an enzyme, eg. diaphorase, to convert NADH back to NAD+, and a quinone, such as benzoquinone, which is added at the same time as the diaphorase and is simultaneously converted to hydroquinone. The reactions of antibody and enzymes are in situ in a standard Rank cell provided with a plasticised PVC/dialysis membrane (as described hereinbefore) and polarised at +500 V (vs Ag). Only benzoquinone and hydroquinone, being lipophilic, can partition into, and hence permeate through, the PVC so that only these components enter the electrolyte associated with the electrode system. Of these two components, only hydroquinone is electroactive at the electrode and the amount of hydroquinone measured electrochemically in turn provides a measure of the amphetamine in the original sample.

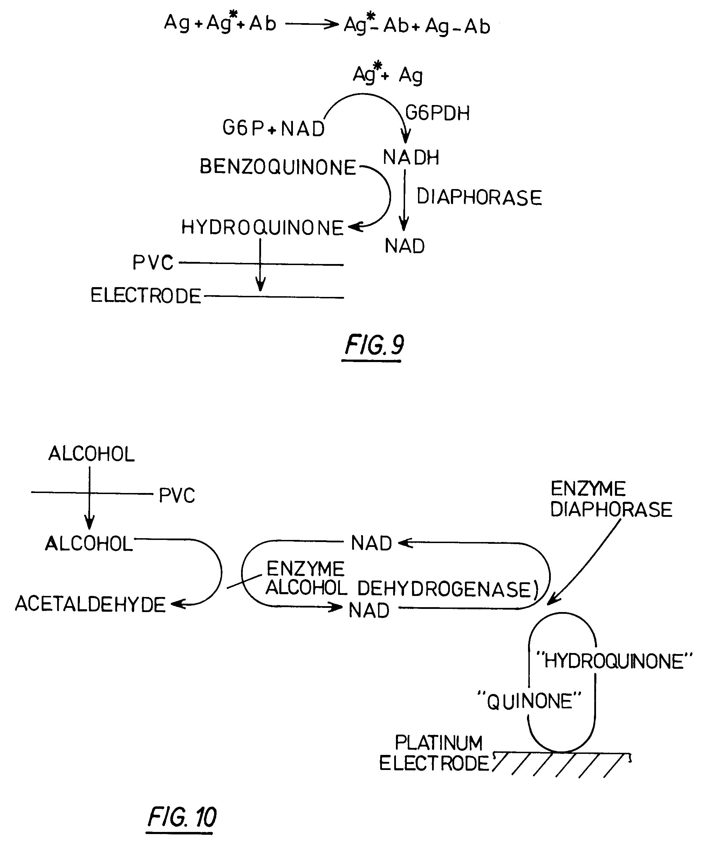

The use of the PVC barrier means that there is no interference from the NADH, and no removal of bound Ag* etc is required in the process, thereby affording a simple assay with no interference. The scheme of the assay is shown diagrammatically in Figure 9.

Another proposed application of the invention is in the determination of alcohol in a sample, eg. alcohol in blood samples, and the scheme employed is shown diagrammatically in Figure 10. In this case, the various reagents are introduced beneath the PVC membrane, ie. between the PVC membrane and the electrode system and the device may be of the "dipstick" type described with reference to Figures 7 and 8. The dipstick is contacted with the sample and any alcohol in the sample, being lipophilic, permeates through the plasticised PVC membrane which excludes interferents commonly occurring in physiological samples. The electrolyte between the membrane and the platinum electrode also contains a mixture comprising the enzymes alcohol dehydrogenase and diophorase, NAD and a suitable quinone, as discussed below.

Alcohol permeating through the PVC membrane is converted by the alcohol dehydrogenase to acetaldehyde, simultaneously converting NAD to NADH. The production of NADH results in the conversion of the quinone, in the presence of diaphorase, into a "hydroquinone" (with simultaneous recycling of NADH to NAD) which, being electroactive is detected at the electrode, the electrode reaction serving to recycle the hydroquinone back to the quinone so that overall the starting compounds are continually recovered for re-use.

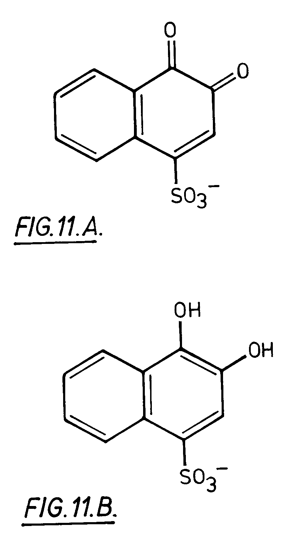

The enzymes diophorase and alcohol dehydrogenase and also NAD and NADH cannot permeate the PVC membrane and are therefore retained in the electrolyte. Quinone and hydroquinone however are both lipophilic and if present in this form would tend to pass through the PVC membrane. Thus, to eliminate loss of these components, it is believed that a modified quinone can be employed which is substantially non-lipophilic both in its original form and when converted to its hydroquinone analogue, thereby allowing these components to be retained within the electrolyte. A suitable modified quinone is 1,2 naphthoquinone-4-sulphonic acid which has an ionisable "tail" ($SO_3^-$), see Figure 11A, and which can be converted to an electroactive and substantially non-lipophilic hydroquinone analogue, see Figure 11B.

## Claims

1. A sensor device comprising detecting means for detecting a preselected lipophilic component (especially one which is electrochemically active) present in a fluid sample and providing an output representative of the content of said component in the sample, a permselective medium and means immobilising said permselective medium as a continuous liquid or gel phase to form a substantially electrically insulating barrier between the detecting means and a fluid sample to be analysed, the permselective medium being substantially impermeable to charged substantially non-lipophilic species that may be present in samples to be analysed and being selective, by partitioning, for lipophilic species whereby any of said pre-selected lipophilic component in the sample partitions into and diffuses through the barrier for detection on the other side of the permselective medium barrier by the detecting means, and preferably including also an electrolyte medium, separated by the permselective medium barrier from the fluid sample to be analysed.

2. A sensor device as claimed in Claim 1 wherein the detecting means is of an electrochemical nature (for example an electrode which is preferably of the non-potentiometric type and especially an ion-selective electrode) and there is present, between said permselective medium barrier and said electrode, an electrolyte medium containing an agent which reacts with at least a preselected lipophilic compound to produce an ionic species, or a concentration or activity change in an ionic species, to which the electrode is responsive.

3. A sensor device as claimed in Claim 1 or Claim 2 wherein the permselective medium is immobilised by confinement between a pair of supports.

4. A sensor device as claimed in any of Claim 1 to 3 wherein the permselective medium is immobilised by incorporation in a polymeric material (especially poly vinyl chloride) such that the permselective medium forms a continuous phase and is preferably present throughout the thickness of the layer or film formed by the polymeric material and the permselective material, especially as a plasticiser which is substantially miscible with the polymeric material so as to form a substantially homogeneous phase in which the plasticiser is immobilised by the polymeric material.

5. A sensor device as claimed in Claims 1 to 4 which comprises means, for example an enzyme, for interacting with the preselected component to produce a directly detectable electrochemically active species, for example small uncharged neutral hydrophilic compounds, especially hydrogen peroxide.

6. A method for determining a lipophilic analyte in a fluid specimen, which uses a sensor device as claimed in any of Claims 1 to 5 and 10.

7. A method for determining a lipophilic analyte as claimed in Claim 6, which comprises contacting the sample with a permselective medium which is immobilised as a continuous liquid or gel phase to form an electrically insulating barrier layer enclosing a detecting system including an electrolyte medium, such contact of the specimen with the permselective material being made at the opposite side of the barrier to that side presented towards the electrolyte medium and the permselective medium being substantially impermeable to said charged substantially non-lipophilic species and being selective, by partitioning, for said lipophilic analyte, and using the detecting system to directly or indirectly detect the lipophilic analyte diffusing through the barrier.

8.  A method as claimed in Claim 7 adapted for the electrolytic determination of hydrogen peroxide derived from a specimen containing charged substantially non-lipophilic species, (especially hydrogen peroxide which is generated as a result of interaction of a desired component with an enzyme, for example glucose oxidase which is used to promote the oxidation of glucose in the presence of oxygen) which method comprises contacting the specimen with a permselective medium which is immobilised as a continuous liquid or gel phase to form an electrically insulating barrier layer enclosing a detecting system including an electrolyte medium, such contact of the specimen with the permselective material being made at the opposite side of the barrier to that side presented towards the electrolyte medium and the permselective medium being substantially impermeable to the said charged substantially non-lipophilic species and being at least partially selective towards hydrogen peroxide, and using the detecting system to detect hydrogen peroxide diffusing through the barrier.

9.  A method as claimed in Claims 6 to 8 wherein the pH values on opposite sides of the barrier are adjusted to optimise the signals produced by the electrode system and/or control the lipophilicity of components in the sample.

10. A sensor or a method for determining a lipophilic analyte or hydrogen peroxide, substantially as described with reference to the accompanying Examples.

_FIG.1_

_FIG. 2_

*FIG. 3*

*FIG.4*

*FIG.5*

*FIG.6*

FIG 8

FIG.7

$$Ag + Ag^* + Ab \longrightarrow Ag^*{-}Ab + Ag{-}Ab$$

$$Ag^* + Ag$$

G6P + NAD

G6PDH

BENZOQUINONE

NADH

DIAPHORASE

HYDROQUINONE

NAD

PVC

ELECTRODE

*FIG. 9*

ALCOHOL

PVC

ENZYME
DIAPHORASE

ALCOHOL

NAD

ENZYME
(ALCOHOL DEHYDROGENASE)

ACETALDEHYDE

NAD

"HYDROQUINONE"

"QUINONE"

PLATINUM
ELECTRODE

*FIG. 10*

14

FIG.11.A.

FIG.11.B.

## EUROPEAN SEARCH REPORT

European Patent Office

Application Number

EP 92 30 2131

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | WO-A-9 005 910 (I-STAT CORPORATION)<br>* page 53, line 16 - page 57, line 18 *<br>* page 119, line 14 - page 120, line 28 *<br>* page 130, line 27 - page 132, line 11 *<br>* claims 3,5,35,36; table 2 *<br>--- | 1-10 | C12Q1/00<br>G01N33/94 |
| Y | US-A-4 236 987 (J.G. SCHINDLER ET AL.)<br>* claim 1 *<br>--- | 1-10 | |
| T | BIOCHIMICA ET BIOPHYSICA ACTA<br>vol. 1115, 1 November 1991, AMSTERDAM NL<br>pages 157 - 165;<br>M.F. ROSENBERG ET AL.: 'A liposomal enzyme electrode for measuring glucose.'<br>* the whole document *<br>----- | 1-10 | |
| | | | TECHNICAL FIELDS SEARCHED (Int. Cl.5 )<br><br>C12Q<br>G01N |

The present search report has been drawn up for all claims

| Place of search | Date of completion of the search | Examiner |
|---|---|---|
| THE HAGUE | 15 JUNE 1992 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)